# EUROPEAN PATENT APPLICATION

(11) **EP 3 705 143 A1**
(43) Date of publication of application: **09.09.2020**
(21) Application number: 19160452.9
(22) Date of filing: 04.03.2019
(51) Int. Cl.: A61L 31/06, A61L 31/14, A61L 31/00

(54) **BIODEGRADABLE MESH IMPLANT FOR SOFT TISSUE REPAIR, IN PARTICULAR HERNIA REPAIR**

(71) Applicant: Baer, Hans U., 8807 Freienbach (CH)
(72) Inventor: Baer, Hans U., 8807 Freienbach (CH)
(74) Representative: Schaad, Balass, Menzl & Partner AG

(57) **Abstract**

The present invention relates to a biodegradable mesh implant for use in soft tissue repair, in particular surgical hernia or fistula repair, within the body of a patient. The mesh implant comprises a porous, hydrophilic biodegradable polymeric carrier mesh 10 having a water contact angle of less than 75° and being made of at least a first polymer comprising polylactic acid, and fibroblasts 16 on or within the polymeric carrier mesh.

## Description

The present invention relates to a biodegradable mesh implant comprising a polymeric carrier mesh seeded with fibroblasts for use in soft tissue repair in general and in particular for surgical hernia repair as claimed in Claim 1. The present invention further relates to a kit comprising the inventive mesh implant and an additional polymeric support mesh.

Wound healing is a complex process that involves an orchestration of a series of interrelated cellular events and cytokine cascades. The main phases of wound healing include fibrin clot formation, followed by infiltration of inflammatory cells and fibroblasts, generation of granulation tissue and angiogenesis, and re-epithelialization. An important role play growth factors and cytokines that are first supplied by degranulating platelets and later by fibroblasts and inflammatory cells, in particular neutrophils and macrophages. Due to its complex nature, the wound healing process is also fragile and susceptible to interruption or failure leading to the formation of chronic wounds whose healing is seriously impaired. Therefore, depending on the size and type of the soft tissue defect, surgical repair may be required to aid the wound healing process and lower the risk for complications.

Within the field of surgical soft tissue repair, use is often made of a mesh implant consisting of a resorbable or non-resorbable material that is inserted to cover the area of the tissue defect. The mesh implant is used as mechanical closure of the defect and to provide a support structure for the regenerating tissue, i.e. for ingrowing cells that form a strong scar fibrous tissue around the mesh implant.

Apart from chronic wounds, mesh implants are often used in surgical repair of hernial defects, in particular in the abdominal wall, which may be a result from trauma, tumour resection, or prolapse. In general terms, a hernia can be described as a passage of organs or organ parts out of the natural body cavity through a preformed or acquired gap. The most frequently encountered hernia types are inguinal hernia or femoral hernia, hiatal hernia, umbilicial hernia and incisional hernia, the latter being a hernia that pushes through a past surgical incision or operation. In surgical hernia repair the mesh implant is inserted to cover the area of the defect in the abdominal wall to achieve tension free closure - with or without sewing together the surrounding muscles and fascia. This can be done under local or general anesthesia using an open incision technique or a laparoscope in a minimally invasive procedure.

The reasons why hernias occur are not yet fully understood. One suggested theory in the field is that some patients, due to collagen metabolic disorders, have a genetic predisposition for developing recurrent hernias, especially in older patients. An altered ratio of collagen types I and III in these patients, with an increase in collagen type III, is believed to reduce the mechanical strength of connective tissues. It seems that decreased tensile strength of collagen type III plays a key role in the development of incisional hernias, see KLINGE, U. et al. Abnormal collagen I to III distribution in the skin of patients with incisional hernia. Eur Surg Res. 2000, vol.32, no.1, p.43-48*.* A disorder in collagen production is also believed to be a contributing factor to the development of chronic wounds, i.e. wounds that do not heal properly and open again and again, especially in diabetic patients and patients with vascular disorders.

The commercially available meshes used in surgical soft tissue repair today are either non-degradable or are fully degradable and absorbed within the patient's body after a certain time.

Non-absorbable mesh implants are most often made of various plastics, which are known to stay biostable and safe for a number of years after implantation. However, introducing a foreign material into the human or animal body can be accompanied with side effects like mesh migration, chronic inflammation, risk of infection etc. The introduction of a relatively large plastic piece into the body may also induce a foreign body-reaction caused by the body's immune defence system. As a result, the mesh implant may crumple up and loose its tissue supporting function. A further problem is that the plastic materials used for the meshes, e.g. polypropylene, show a limited cell-growth stimulating effect. Therefore, ingrowth of cells into the defect is often limited and wound healing is prolonged.

Fully absorbable (biodegradable) meshes, on the other hand have the advantage that there is no foreign tissue that remains within the body. Some fully biodegradable materials, such as poly-lactic acid (PLA) or collagen have shown to promote fibrogenesis, i.e. ingrowth of cells into the soft tissue defect, which is important for rapid and sustained wound healing. Nevertheless, if the mesh is degraded and absorbed too fast, the scar plate that is gradually built over the tissue defect will not yet be able to withstand stresses applied to the repaired soft tissue during daily activities. This is of particular importance in case of hernia repair, since the scar plate covering the defect must withstand the intraabdominal pressure, otherwise, a hernia relapse is almost guaranteed. In addition, meshes made from e.g. PLA have the disadvantage of being rather brittle and tend to break if folded or rolled to introduce the mesh though a laparoscopic tool.

The problem solved by the present invention is therefore to provide a mesh implant that allows for rapid, safe and stable closure of soft tissue defects, in particular chronic wounds, fistulas or hernia defects. At the same time, the components of the mesh implant shall be easy and inexpensive to manufacture and is usable by employing conventional open and laparoscopic surgical methods.

This problem is solved by the mesh implant according to Claim 1 and the implant kit according to Claim 15. Preferred embodiments are subject of the dependent claims.

In line with the present invention, a mesh implant in soft tissue repair, in particular surgical hernia or fistula repair, within the body of a patient is provided. The implant comprises a porous, hydrophilic biodegradable polymeric carrier mesh made of at least a first polymer comprising polylactic acid. The carrier mesh has a water contact angle of less than 75° which means that it is hydrophilic. The implant of the present invention further comprises fibroblasts that are present on or within the polymeric carrier mesh.

One key element of the present invention is that the polymeric carrier mesh carries fibroblasts. It has surprisingly been found that the formation of new tissue, especially scarring tissue, to close a hernia defect can be evoked by bringing additional fibroblasts (of any origin, e.g. autologous, heterologous, xenophilic) into the area of the defect. Thus, instead of focusing on promoting tissue ingrowth of e.g. smooth muscle cells and fibroblasts from adjacent tissue into the soft tissue defect, e.g. into a chronic wound or a hernia, the implant of the present invention uses the degradable carrier mesh to bring an augmented number of fibroblasts into the defect area. The fibroblasts help constructing extracellular matrix tissue and various types of collagen fibres, thereby forming a scar plate closing the soft tissue defect. As such, the scar plate will form a firm connection between the degradable (and thus temporary) polymeric carrier mesh and the rims of the defect as well as the other surrounding structures (e.g. the abdominal wall in case of an abdominal hernia) to ensure the desired support function by creating additional cicatrisation.

Within the present application, the following definitions apply:
The term "biodegradable" means that the mesh can be absorbed over time if placed within a living organism, in which it is surrounded by body fluids.

The term "mesh", as used throughout this application, refers to a three-dimensional support, i.e. a matrix, meaning a scaffold- or sponge-like structure, which is suitable for being colonized by cells. In this sense, the mesh serves as a three-dimensional template which can be colonized by cells or tissue. This colonization can take place *in vitro* or *in vivo.* Furthermore, the mesh serves, in connection with transplantations, for locating the transplant and also as a place holder for tissue which is gradually formed *in vivo.*

The term "porous" thereby refers to a structure comprising pores, i.e. cavities or void regions. These pores may have a round shape and/or an angular shape in a 2-dimensional section and/or a canted shape when seen 3-dimensionally. The shape of the pores may also be characterized by extensions such that it can be compared with the shape of nerve cells. As such, the term "pores" also refers to cavities formed by filaments enclosing a void region. These pores or cavities may also be interconnected, meaning that the pore walls between two adjacent pores can comprise holes, forming a connection between said adjacent pores. In this regard, it is to be noted that the pores can also be formed by the void regions between the granules of a hydrogel.

Within the meaning of this application the term "hydrophilic or "hydrophilicity" as used in the context of the present invention refers to a water contact angle of the hydrophilic surface area directly on the carrier mesh being less than 90°. In particular, it refers to a contact angle of less than 75°, preferably less than 60°. The hydrophilic surface can be obtained for example by increasing the content of PLLA in the material composition and/or by post-processing methods, such as plasma-treatment (i.e. treatment with an ionized gas plasma) and/or covering the surface with a natural polymer, such as gelatin. More details in this respect are given in the experimental section further below.

The term "biocompatible polymers" refers to polymers which are biologically tolerated and do not cause rejection when brought into a living organism. For the present invention, biocompatible polymers also encompass polymers which are recognized by a host as being foreign but whose rejection can be suppressed by appropriate immunosuppression.

The expression "biodegradable" refers to a material which can be converted into metabolizabled products in living organisms (or body fluids or cell cultures derived from living organisms). Biologically degradable polymers include, for example, polymers which are bioresorbable and/or bioerodable. "Bioerodable" denotes the ability to be soluble or suspendable in biological liquids. Bioresorbable means the ability to be able to be taken up by cells, tissues or fluids of a living organism.

In a preferred embodiment, the polymeric carrier mesh has a water contact angle of less than 40°, preferably less than 25°, more preferably less than 15°, Most preferably, the water contact angle of the hydrophilic surface of the carrier mesh is within the range of 0° to 10° and is thus "super hydrophilic".

The term "contact angle" as used in the context of the present invention relates to the contact angle of water on a surface, i.e. to the angle formed at the interface where water meets the surface. Thereby, "water" used for the contact angle measurement relates to pure water, specifically ultrapure water. In particular, the contact angle measurement is carried out by the sessile drop method (e.g. by means of a device of the type EasyDrop DSA20E, Kruss GmbH) using a drop size of 0.3 µl. Contact angles are generally calculated by fitting a circular segment function to the contour of the droplet placed on the surface.

It has been found that a mesh having hydrophilic properties promotes tissue ingrowth of e.g. smooth muscle cells and fibroblasts from adjacent tissue of the hernial defect. In addition, the porous nature of the carrier mesh provides a growth-stimulating environment for the cells that help constructing extracellular matrix tissue and various types of collagen fibres, thereby forming a scar plate closing the hernia. The formation of the scar plate will establish a firm connection between the degradable (and thus temporary) polymeric carrier mesh and the rims of the hernia defect as well as the abdominal wall or other surrounding structures. While the degradation of the carrier mesh continues, the newly formed scar tissue will provide necessary support function by creating additional cicatrisation, thereby preventing recurrent hernia formation. In the end, after complete degradation of the carrier mesh, i.e. when the polymeric components of the mesh have been absorbed, no permanent foreign material will be left within the patient's body.

In a preferred embodiment, the carrier mesh has a flat sheet-like shape and is elastically deformable to allow folding or rolling thereof. In this embodiment, it is preferred that the carrier mesh is elastically deformable, such it can be folded or rolled and it can return to its original shape. This allows insertion of the rolled or folded mesh through a trocar in a laparoscopic procedure.

If the carrier mesh is provided in a sheet-like shape, it has preferably a thickness of at least 0.1 mm, more preferably at least 0.3 mm, further preferably from about 1 mm to about 4 mm, in some cases it may be even thicker, i.e. up to 10 mm.

If provided in sheet-like shape, the cross-section of the carrier mesh can be of any kind, for example rectangular, square, circular, oval, etc., and can then be cut to suit the shape of the hernia defect. For example, the overall shape of the cross-section can be circular or oval.

The carrier mesh may be in the form of one single continuous structure or it may also be provided in form of multiple smaller mesh pieces (i.e. "patches") that are separate from one another. These small mesh patches may be contained in a paste-like, liquid or semiliquid formulation.

In an alternative preferred embodiment, the carrier mesh is in form of a hydrogel - i.e. without a specific three-dimensional outer or cross-sectional shape - A hydrogel, because of its hydrophilicity, aids the growth of tissue into the pores, whereby immediate anchorage of the carrier mesh to the tissue is achieved. The hydrogel may also be mixed with further substances, such as growth factors or pharmaceutically active substances.

As regards the material composition of the carrier mesh it is preferred that the first polymer consists to at least 70% of poly(lactic acid), more preferably of at least 80% poly(lactic acid). In particular if the mesh implant is intended for use in open surgery, the first polymer preferably essentially consists of poly(lactic acid). PLA is preferred since it increases the hydrophilic properties of the carrier mesh. The term "poly lactic acid" (PLA) includes, for example, poly-L-lactic acid (PLLA) or poly-D,L-lactic acid (racemic mixture of L- and D-lactides; PDLLA). A specific example of a preferred first polymer is Poly(L-lactide) that is commercially available from Sigma Corporation (PLLA catalogue number P1566) with a molecular weight of 85,000-160,00. A suitable alternative is PLLA from Durect Corporation (Lactel® catalogue number B6002-2).

Although it is preferred that the first polymer essentially consists of pure PLA, it may also be made from a mixture of PLA with other biodegradable materials, such as PGA. One reason is that pure PLA meshes are less strain-resistant compared to meshes made of PLA mixed with other polymers, such as PGA. PGA is the abbreviation of poly-glycolic acid. Co-polymers of PLA and PGA - so-called poly-lactide-co-glycolic acid (PLGA or PLG) - are attractive candidates for fabricating biodegradable meshes due to their flexible and well-defined physical properties and relative biocompatibility. Additionally, their degradation products are low molecular weight compounds that enter normal metabolic pathways. Furthermore, copolymers of PLGA offer the advantage of a large spectrum of degradation rates from a few days to years by simply varying the copolymer ratio of lactic acid to glycolic acid.

In particular if the mesh implant is intended to be used laparoscopically in minimally invasive surgery, it must be insertable through a laparoscopic tool, such as a trocar. For these applications, the carrier mesh is preferably in form of a paste or hydrogel or alternatively in a shape that can be elastically folded or rolled for insertion through a laparoscopic tool. Elastic properties can be provided by using PLGA, i.e. a mixture of PLA and PGA, as the first polymer.

Thus, for a foldable carrier mesh, the first polymer is made from PLGA, specifically a poly(glycolic acid-lactic acid) mixture having a lactic acid content of about 50 mol% and a glycolic acid content of about 50 mol%). Such a 50:50 poly(D,L-lactide-co-glycolide) material can be purchased, for instance, as RESOMER® RG 502 from Evonik Industries AG (Essen, Germany) or from Durect company (Cupertino, CA, USA). Further preferred polymer mixtures are poly(D,L-lactide-co-glycolide) 65:35, e.g. RESOMER® RG 653; poly(D,L-lactide-co-glycolide) 75:25, e.g. RESOMER® RG 752 or Sigma Corporation PLGA catalogue number P1941; poly (D, L-lactide-co-glycolide) 85:15, e.g. RESOMER® RG 858 or LACTEL® Absorbable Polymers.

Preparation methods for preparing porous meshes from these synthetic polymers are well known in the art. One possibility is the use of a salt-leaching technique, as described, for instance in EP 2256155.

In a preferred embodiment, the carrier mesh essentially consists of only the first polymer alone or of the first polymer and a further natural polymer selected from the group consisting of collagen, gelatin, laminin, fibrinogen, albumin, chitin, chitosan, agarose, hyaluronic acidalginate and mixtures thereof. This way, the carrier mesh will provide a particularly hydrophilic, cell-friendly environment, which increases the survival and proliferation rate of cells on and within the mesh.

As regards the degradation of the carrier mesh within the body - which will usually occur through bio-absorption of its components - it is preferred that it has a total degradation time in a living organism of 1 to 12 months, preferably 1 to 8 months, more preferably about 1 to 6 months, most preferably about 2 to 4 months.

As mentioned, the carrier mesh may comprise or be at least partially covered with at least one natural polymer selected from the group consisting of collagen, gelatin, laminin, fibrinogen, albumin, chitin, chitosan, agarose, hyaluronic acidalginate and mixtures thereof, whereby collagen and laminin are preferred.

From the above-mentioned natural polymers, collagen is most preferred. Collagen is a biomolecule of the extracellular matrix (ECM) and the major component of skin and bone. Thanks to its nano-fibrous architecture collagen is particularly effective in promoting cell adhesion, growth and differentiated function in tissue cultures. However, it has also been found that if the natural polymer comprises collagen, the hydrophilic properties of the carrier mesh according to the present invention are particularly enhanced. In this regard, the term "collagen" as used in the context of the present invention encompasses naturally derived collagens and synthetically produced collagens and also collagen derived substances, such as gelatine, which is a hydrolysed form of collagen.

In a preferred embodiment, the natural polymer essentially consists of collagen. In this regard, the natural polymer may consist of only one type of collagen, i.e. type I, or may consist of a mixture of collagen types, e.g. a mixture of type I collagen and type IV collagen. In the latter case, preference is given to the mixture containing the proteins in approximately equal percentages by weight. Collagen type I is most preferred, since is readily available, has a high degree of biocompatibility and provides additional tensile strength. In addition, it is one of the main components of natural blood vessels and provides a natural attachment site for cells involved in the wound healing process. Finally, the degradation product of collagen type I to III have also been shown to induce a chemotactic attraction of human fibroblasts, which is particularly beneficial for the intended use of the carrier mesh as an implant comprising fibroblasts.

Another advantage of the inventive implant is the ability to have agents incorporated into the carrier mesh and subsequently delivered to the wound site which affect cell growth, such as collagen types IV and V, fibronectin, laminin, hyaluronic acid, and proteoglycans. Similarly, pharmacologically active agents such as epidermal growth factor, platelet derived growth factor, transforming growth factor beta, angiogenesis factor, antibiotics, antifungals, spermicidals, hormones, enzymes, and/or enzyme inhibitors can also be incorporated into the carrier mesh.

To promote scar tissue formation it is therefore preferred that the carrier mesh further comprises growth factors, preferably selected from the group consisting of interleukins, acidic fibroblast growth factor, basic fibroblast growth factor, epidermal growth factor, insulin like growth factor, insulin like growth factor binding protein, platelet-derived growth factor, transforming growth factor alpha, transforming growth factor beta, VEGF, and HGF. These growth factors are important for regulating cell proliferation and differentiation, protein synthesis and ECM (extracellular matrix) remodelling. In particular, b-FGF, PDGF, VEGF, and HGF have shown to increase granulation, epithelialisation and capillary formation through angiogenic cytokines secretion. They have also proven to inhibit neutrophil and macrophage migration to wound location by secreting factors that inhibit migration and both IL-1α and IL-1β suppression, and to secrete anti-inflammatory factors which prevent apoptosis and improve wound healing.

Preferably, the growth factors are added to the carrier mesh together with a placental mesenchymal secretome. One preferred commercially available secretome is a secretome comprising stem cells from human Wharton's Jelly Stem Cell (CM-hWJSC) that were cultured in hypoxia condition. This stem cell secretome can be obtained from Stem Cell and Cancer Institute (PT. Kalbe Farma Tbk.) The secretome is used to stimulate the growth of implanted cells on the carrier mesh. In view of its use as medical device, the addition of the secretome can be employed extracorporeal in the incubator to stimulate growth of the cells before implantation. As soon as sufficient cell growth has been achieved, the mesh can be washed with media prior to implantation.

The use of secretome comprising stem cells from human Wharton's Jelly Stem Cell (CM-hWJSC) that were cultured in hypoxia condition to stimulate cell growth has proven to be highly effective not only for the stimulation of fibroblasts, but also for other cells, in particular cells that are more delicate to handle and to cultivate, such as liver cells (hepatocytes). In fact, hepatocytes are generally co-cultivated with cells of the pancreas, specifically cells of Islets of Langerhans, as this has shown to increase the survival and proliferation rate of the hepatocytes. When adding the mentioned secretome to hepatocytes in culture media, growth and proliferation of the hepatocytes has found to be even better than in cocultivation with cells of Islets of Langerhans.

The fibroblasts provided on the carrier mesh are preferably autologous fibroblasts of the patient that is intended to receive the implant. This way, the risk of the patient showing an undesired immune response due to the implantation of foreign cellular material is greatly reduced.

The present invention also relates to a method for preparing an implant as described above by the following steps:
a) providing a biodegradable polymeric carrier mesh comprising polylactic acid;
b) sterilizing the polymeric carrier mesh by treating it with H₂O₂ at a temperature below 50°C, preferably below 40°C; and
c) inoculating the biodegradable polymeric carrier mesh with fibroblasts.

The polymeric carrier mesh is preferably of the type described in the above sections of this application. As such, it preferably consists of a first degradable polymer, which includes polylactic acid and optionally a further natural polymer. The first degradable polymer may consist of PLA or it may be mixture of PLA and another synthetic polymer, such as poly(lactide-co-glycolide) (PLGA). The natural polymer is preferably selected from the group consisting of collagen, gelatin, laminin, fibrinogen, albumin, chitin, chitosan, agarose, hyaluronic acidalginate and mixtures thereof, whereby collagen is most preferred. The carrier mesh may also be provided as a two-layered structure, e.g. a structure that comprises a PLA layer on top of a PLG layer.

The sterilization step b) of the inventive method is a key step in the preparation of the implant of the present invention. It has been surprisingly found that a sterilization using hydrogen peroxide allow for perfect sterilization of the carrier mesh, without using any of the conventional sterilization techniques involving hot steam, gamma rays, electron beam irradiation or harsh chemical treatments that were shown to cause alteration or even degradation of the molecular structure of the polymeric carrier mesh and therefore not only decreases its mechanical and enzymatic resistance but also reduces its hydrophilic properties. In addition, the inventive hydrogen peroxide sterilization method preserves any collagen layer that may be provided on the carrier mesh. In contrast to the conventional sterilization techniques that lead to denaturalization of collagen layers, in particular in case of nano-thick layers of collagen, the H₂O₂ treatment as described above does have this deteriorating effect.

Preferably, the H₂O₂ treatment is conducted by exposing the carrier mesh to a H₂O₂ containing atmosphere for at least 1 hour, preferably at least 4 hours, more at least 8 hours, most preferably for about 10 to 12 hours. The hydrogen peroxide containing environment can either be provided by H₂O₂ plasma treatment or by placing the substrate to be sterilized into a vacuum chamber, together with a source of (generally liquid) hydrogen peroxide. Upon application of a vacuum inside the vacuum chamber, the hydrogen peroxide sublimes and a hydrogen peroxide-containing atmosphere will be created. In addition, the negative pressure applied in the vacuum chamber is preferably 6 to 12 mbar, more preferably 8 mbar to 10 mbar, most preferably about 9 mbar.

In an alternative preferred embodiment, sterilization can be achieved by the direct effect of reactive gases, specifically hydrogen peroxide gas plasma. If desired, plasma-generated UV light can additionally or alternatively be applied to kill bacteria.

The method of the present invention also encompasses embodiments in which an additional sterilization or cleaning treatment is performed before or after the hydrogen peroxide treatment. In particular, embodiments are encompassed in which, before or after sterilization step b), the carrier mesh is subjected to a plasma treatment using an ionized gas plasma other than H₂O₂, preferably selected from the group consisting of helium, argon, nitrogen, neon, silane, hydrogen, oxygen and mixtures thereof. The plasma treatment preferably involves a low-pressure plasma treatment, in which the carrier mesh is exposed to an ionized gas plasma at a pressure in the range of 10⁻² to 10⁻⁶ bar and a temperature below 50°, preferably below 40°C, for at least 2 hours. Preferably, a plasma treatment using an ionized hydrogen or nitrogen gas plasma is used before the hydrogen peroxide treatment. Preferred treatment times are between 8 and 12 hours, most preferably about 10 hours.

The term "plasma" thereby generally refers to an excited and radicalized gas, i.e. an electrical conducting process gas involving electrons and ions. Plasma is commonly generated by means of electrodes in a vacuum chamber (so-called "RF plasma approach"), but it can also be generated using capacitive or inductive methods, or microwave radiation.

As an alternative to a plasma treatment, the sterilization of the carrier mesh may also be achieved by placing it in a H₂O₂-containing environment at a temperature below 50°C, preferably below 40°C, for at least 2 hours. The H₂O₂-containing environment can be created within a vacuum chamber, by placing the carrier mesh into the chamber, together with an open flask or dish containing a H₂O₂ solution and by subsequently evacuating the chamber to a negative pressure that leads to evaporation of the H₂O₂. It is preferably in the range 10⁻⁶ to 10⁻² bar, preferably 10⁻³ to 10⁻² bar. Specifically preferred are pressures of 6 to 12 mbar, e.g. about 9 mbar. The treatment time depends highly on the pressure within the chamber and the concentration of the H₂O₂ solution. Preferably, the H₂O₂ solution comprises H₂O₂ in an amount of about 30% by volume or less. Preferred treatment times are at least 4 hours, more preferably between 8 and 12 hours, most preferably about 10 hours.

To aid the wound healing process, the above-described method preferably includes an additional step in which a secretome comprising growth factors, in particular placental mesenchymal secretome, is given on or into the carrier mesh.

The present invention also relates to the use of the inventive implant in primary wound closure, e.g. after a primary abdominal incision, to aid wound healing, in particular in case of chronic wounds, and scarring processes. In addition, it is also highly suitable for use in the closure of fistulas or hernias.

In a further aspect the present invention also relates to the method of closing a hernia using the implant described above. In general, during said method, the degradable polymeric carrier mesh with the seeded fibroblasts will be placed above the defect, e.g. the abdominal wall (inguinal hernia repair according to Lichtenstein), beneath the muscle layer of the abdominal wall (sub-lay technique), as an IPOM repair or in any technique a surgeon may see fit to repair.

Apart from hernias, the mesh implant of the present invention is also particularly well suited for surgical closure of fistulas. A fistula is a connection lined with epithelial tissue pathologically connecting the intraabdominal space with the skin having an opening at the skin level - often producing fluid leaking through the skin - or a pathological connection between the large or small bowel to the anal area. Fistulas are difficult to treat surgically and are prone to repeated recurrences. Until today, complete resection of the entire fistula is the therapy of choice. Various materials have been developed to be inserted into the resected channel to improve the healing process. None has proven to guarantee long-time success. However, it has been found that insertion or implantation of the carrier mesh carrying fibroblasts as described above substantially increases the healing process. This effect was also observed in chronic wounds and fistulas. In this regard, in line with the description above, the carrier mesh can be sheet-shaped or without defined geometry in form of a paste or hydrogel. The present invention further relates to a kit for use in surgical soft tissue repair, in particular fistula or hernia repair, within the body of a patient, wherein the kit comprises a) a polymeric carrier mesh as defined above and b) an additional, preferably biodegradable, support mesh having a slower degradation rate than the polymeric carrier mesh.

If the support mesh is biodegradable, it has preferably a degradation rate within the range of 4 to 12 months.

The non-degradable or degradable support mesh is intended to be placed beneath the polymeric carrier mesh to bridge the hernia after the polymeric carrier mesh has degraded. This means that in general, the support mesh will provide an additional support function after the first three months.

In a preferred embodiment, the support mesh is biodegradable and will have degraded after 12 months at most. Similar to the carrier mesh, the support mesh is preferably at least partly hydrophilic and thus promotes the formation of the scar plate bridging the hernia.

Most preferably, the support mesh is a common commercially available mesh.

In a preferred embodiment, the polymeric support mesh comprises one or more of the polymers selected from the group consisting of poly(glycolic acid), poly(lactic acid), poly(glycolic acid-lactic acid) and mixtures thereof. Also, in this embodiment, if the polymeric carrier mesh is biodegradable, then it has a faster degradation rate than the support mesh.

While the support mesh is said to have a slower degradation rate than the carrier mesh, it may also be that portions of the support mesh have a degradation rate that is similar to the one of the carrier mesh. For instance, in the embodiment, in which the support mesh has a top and a bottom layer as defined in the above paragraph, the top layer may have a faster degradation rate than the bottom layer, such that e.g. the degradation time for the top layer in the living organism is between 1 and 3 months, preferably about 2 months, and the degradation time for the bottom layer in the living organism is between 4 and 8 months, preferably about 6 months. This means that in this embodiment, it is mostly the bottom layer of the support mesh that provides an additional support function during the first four to eight months. After this time, the bottom layer will preferably have degraded as well.

In a further preferred embodiment, the kit includes an additional two-layered anti-adhesion mesh that comprises two individual or integral layers consisting of
i) a hydrophilic layer facing the peritoneum or exposed abdominal muscle tissue or fascia and having a water contact angle below 60° and
ii) a hydrophobic layer having a water contact angle above 90°.

This embodiment of implant kit provides two major benefits: on the one hand, the polymeric carrier mesh provides a temporary closure of the defect, in particular a hernia or fistula, while the fibroblasts form new scar tissue that will take over the support function of the carrier mesh after its degradation. The two-layered anti-adhesion mesh on the other hand has a top layer with hydrophilic properties that will promote tissue growth on the side facing the soft tissue defect, in particular proliferation of the implanted fibroblasts that help forming the scar plate eventually closing the defect, and a bottom layer with hydrophobic properties that prevent the attachment of various cells, inter alia inflammatory cells, fibrin or debris, to the anti-adhesion mesh from the intraperitoneal side, i.e. the side of the support mesh facing away from the defect, such that the occurrence of inflammation and the formation of adhesive tissue growth between the anti-adhesion mesh or the newly-formed scar plate and the underlying abdominal tissues.

In view of their use in hernia or fistula repair, it is preferred that at least one, preferably all, of the polymeric carrier mesh, the support mesh and the anti-adhesion mesh has/have a flexibility that allows its/their insertion into the patient's body through a laparoscopic tool.

Each of the polymeric carrier mesh, the support mesh and the anti-adhesion mesh can be supplied in any shape whatsoever, for example rectangular, square, circular, oval, etc., and can then be cut to suit the shape of the hernia defect. For example, the overall shape of the mesh can be circular or oval. Alternatively, the mesh can have a generally square shape or a rectangular shape.

When used to close a hernia, the implant kit of the present invention can be used as follows:
First, an implant kit as described above is provided. Then, an incision is made through skin and tissue of a patient to access the defect in the peritoneum. The polymeric support mesh is then placed either above the defect, e.g. the abdominal wall (inguinal hernia repair according to Lichtenstein) or alternatively beneath the muscle layer of the abdominal wall (sub-lay technique) or as an IPOM (intraperitoneal on lay mesh) below the peritoneum before placing the polymeric carrier mesh on top of the support mesh. If provided in sheet-like shape, the carrier mesh may be additionally attached to the abdominal muscles. Then, the incision is closed.

The hernial defect will usually be closed by a scar plate within 3 to 6 months, in which time the biodegradable carrier mesh will gradually lose its strength and consistency. In the end, no permanent foreign material will be left when the components of the mesh have bioabsorbed within the body.

### Experimental Data

### Preparation of the carrier mesh

Sodium chloride (NaCl) particulates were ground using mortar and pestle before being sieved to obtain NaCl particulates ranging from 355 to 425 µm. 9 g NaCl particulates were put in a centrifuge tube and dried in a desiccator. The NaCl particulates were then put into an aluminium pan.

A PLLA solution prepared of 1 g of PLLA pellets dissolved in 5 ml of chloroform was mixed with the NaCl particulates and the mixture was then spread evenly in the aluminium pan to form a flat PLLA layer. The solvent was allowed to evaporate by air-drying of room temperature for a time of 20 to 48 hours, preferably 24 to 36 hours, most preferably 28 to 32 hours. Alternatively, the solvent vaporization was carried out in a controlled temperature oven, placed in a fume hood to extract chloroform toxic gases.

The pre-dried PLLA-NaCl layer was detached from the aluminum pan and dried in a vacuum chamber with a negative pressure of 0.1 MPa for 3-4 days.

The resulting dried PLLA-NaCl layers were put in a beaker and the NaCl particulates were leached out by washing the matrices with deionized water. To this end, the matrices were immersed in ddH₂O (twice deionized water) and kept in a linear shaking bath at 25°C (room temperature), at 60 rpm for 48 hours. The water in the beaker was exchanged every 1-2 hours. The thus prepared PLLA meshes were removed from the beaker and dried in the fume hood overnight.

### Post-processing with collagen

The PLLA meshes were cut into round shape with diameter 1 cm. Each PLLA mesh was then immersed in type I collagen acidic solution (1.0 (w/v)%, pH 3.0, purchased from Wako) under a vacuum so that the pores of the mesh filled with collagen solution. The collagen-PLLA mesh was subsequently frozen at -80 °C for 12 hours and lyophilized under a vacuum of < 5 Pa for an additional 24 hours to remove any solvent from the mesh.

If a cross-linking agent was used (which showed to be non-essential for the preparation of the mesh), the collagen-PLLA mesh was further cross linked by treatment with glutaraldehyde vapour saturated with 25 % glutaraldehyde aqueous solution at 37 °C for 4 hours. After being crosslinked, the mesh was treated with 0.1 M of glycine aqueous solution to block non-reacted aldehyde groups. Instead of glycine, other blocking agents, e.g. casein, albumin, gelatin or methanol-acetic anhydride, may be used. After being washed with deionized water and lyophilized, the polymeric carrier mesh was obtained as the final product. After lyophilization (also known as freeze-drying) the collagen-PLLA meshes were stored in a desiccator until use.

The carrier meshes were prepared with pores having a diameter within the range of 50 to 900 micrometres, preferably 280 to 560 micrometers, most preferably 355 - 425 micrometers.

### O₂ plasma cleaning

Notably, the sterilization method of the present invention also encompasses embodiments in which an additional sterilization or cleaning treatment is performed before or after the hydrogen peroxide treatment. In particular, embodiments are encompassed in which the substrate is subjected to a plasma treatment using an ionized gas plasma other than H₂O₂, preferably selected from the group consisting of helium, argon, nitrogen, neon, silane, hydrogen, oxygen and mixtures thereof. Preferably, a plasma treatment using an ionized hydrogen or nitrogen gas plasma is used before the hydrogen peroxide treatment.

The plasma treatment was conducted within a vacuum chamber for a time of 5 to 20 minutes, preferably 8 to 15 minutes. The treatment parameters were set as follows: Pressure within the vacuum chamber: 0.40 mbar; power: 35 W; oxygen gas flow: 5 sccm (min) - 60 sccm (max).

### Sterilization

Finally, the matrices were subsequently sterilized by placing them in a H₂O₂-containing environment exposing the carrier mesh to a H₂O₂ containing atmosphere as described above with respect to the H₂O₂ treatment of the carrier mesh.

The H₂O₂-containing environment was created within a vacuum chamber, by placing the carrier mesh into the chamber, together with an open flask containing a H₂O₂ solution and by subsequently evacuating the chamber to evaporate the H₂O₂. The H₂O₂ solution comprises H₂O₂ in an amount of 30% by volume. The treatment time depends highly on the pressure within the chamber and the concentration of the H₂O₂ solution. Preferably, the sterilization time was 10 to 12 hours, at a pressure of about 9 mbar.

### Static contact angle measurements, sessile drop method

Contact angle measurements were performed in order to determine the degree of hydrophilicity or hydrophobicity. Usually, the contact angles of the scaffolds were determined by static contact angle measurements. The static contact angles were determined using a sessile drop test with ultrapure water (EasyDrop DSA20E, Kruss GmbH). The water droplets with a size of 0.3 µl were dosed using an automated unit. Values for contact angles were calculated by fitting a circular segment function to the contour of the droplet placed on the surface.

### Fibroblast harvesting, cultivation and seeding

The harvesting of fibroblasts is done by a dermatologic or surgical excision of a full thickness skin specimen under strict sterile conditions of approximately 2 cm times 1 cm. The specimen is placed in a sterile container and covered with pre-warmed PBS containing 2% antibiotic/antimycotic (AB/AM). The specimen is transferred to the laboratory, where it is washed with pre-warmed wash/EGTA solution containing 2% AB/AM. The washed skin specimen is covered with freshly-prepared 200 Units/ml collagenase in complete DMEM media containing 10-20% FBS, 2% AB/AM, 2mM L-glutamin and 1 mM Na-pyruvat solution and then it is incubated - with the dermis-side down - at 37C, 5% CO2 for 3-5 hours. The dermis side is gently scraped with a cell scraper until all dermis part dissolves into collagenase solution. The epidermis part is discarded and the cell suspension is sieved through a 100 µm nylon cell sieve and the cells collected in the sieve are rinsed thoroughly with PBS (2% AB/AM) before being collected in a centrifuge tube. The cells are centrifuged for 10 minutes, the supernatant is discarded and the cells are re-suspended in complete DMEM media containing 10-20% FBS, 2% AB/AM, 2 mM L-glutamin, 1mM Na-pyruvat and 10 mM HEPES. The re-suspended cells are given into a collagen-coated flask and incubated at 37 °C, 5% CO2 for 30 min. Fibroblast cells attached on collagen-coated surface of the flask are separated from non-fibroblast cells that remain in the culture media. Cell counting is performed with trypthan blue staining.

Fibroblasts are incubated in DMEM media if desired. The carrier mesh is seeded with fibroblasts (500'000 cells per 1 cm².

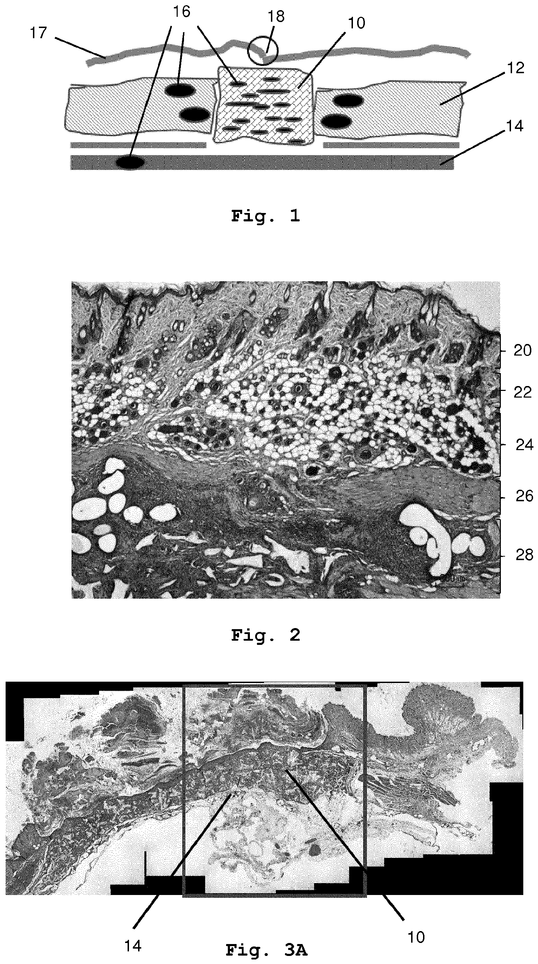

Preferred embodiments with respect to the structure of the inventive implant and its placement within the body of a patient in soft tissue repair are further described in connection with the attached figure, in which
- **Fig. 1**: shows a schematic drawing of a section through a soft tissue defect repaired with the aid of a kit in accordance with the present invention.

The schematic drawing of Fig. 1 shows a defect (gap) in muscle tissue 12 that has been filled with a mesh implant 10 of the present invention. Specifically, a commercially available support mesh 14 was placed beneath the defective muscle tissue 12 before inserting a polymeric biodegradable carrier mesh 10 comprising fibroblasts 16 into the defect. Then the overlaying skin 17 was repaired, i.e. closed by stitches 18. The carrier mesh provides a temporary support structure for the fibroblasts to proliferate and for ingrowth of cells from adjacent tissues. In general, the carrier mesh 10 with the fibroblasts 16 will simply be inserted into or placed over the defect. Thanks to the support mesh 14, displacement of the carrier mesh 10 from the implant site is prevented.

Over the days and weeks after insertion of the mesh implant, the fibroblasts deposited with the carrier mesh within the muscle tissue defect continued to proliferate and spread into the adjacent tissues. This is shown by the fibroblasts that have spread into the adjacent muscle tissue and the support mesh beneath the defect. The foreign fibroblasts have shown to produce collagen, which helps build up scar tissue that firmly closes the defect within the muscle tissue at the time that the carrier mesh has fully degraded.

The inventive mesh implant has also been tested *in vivo* in rats, as will be described in the following sections.

### Histology

An experimental abdominal wall hernia in a rat was repaired with a biodegradable mesh implant in accordance with the present invention, i.e. by means of a carrier mesh carrying fibroblasts. In this experiment, the hernia within the muscle tissue of the abdominal wall was filled with the inventive support mesh carrying numerous fibroblasts and secretome added to the carrier mesh. To keep the implanted carrier mesh in place, two additional support meshes were implanted, one on top and one below the hernia within the muscle tissue and an additional support mesh as part of an inventive kit described above. Histological analysis of the skin wound site is shown in Figs. 2 to 6A:
- Fig. 2: shows a top view on an excised part of an abdominal wall including a formerly hernia area that has been repaired with a kit of the present invention including a carrier mesh carrying fibroblasts and an additional support mesh;
- Fig. 3A: shows a section through the abdominal wall of Fig. 2 along line A, i.e. through the formerly hernia area;
- Figs. 3B, 3C: show enlarged detail sections of Fig. 3A with a magnification of 40x and 200x, respectively;
- Fig. 4A: shows a section through the abdominal wall of Fig. 2 along line B, i.e. adjacent to the formerly hernia area;
- Fig. 4B: shows an enlarged detail section of Fig. 4A with a magnification of 40x;
- Fig. 5A: shows a section through the abdominal wall of Fig. 2 along line C, i.e. at a distance to the formerly hernia area;
- Fig. 6A: shows an enlarged detail section with a magnification of 40x through an abdominal wall of a comparative example, in which a hernia area in was repaired by means of a support mesh without fibroblasts;

As shown in Fig. 2, The abdominal wall includes layers of epidermis 20, dermis 22, subcutaneous tissue 24 with adipose cells, muscle tissue 26 and, within the muscle area 26, a former hernia area 28, in which the carrier mesh and support mesh had been implanted. Thus, a former gap in the muscle tissue 26 was filled with the carrier mesh (and fibroblasts).

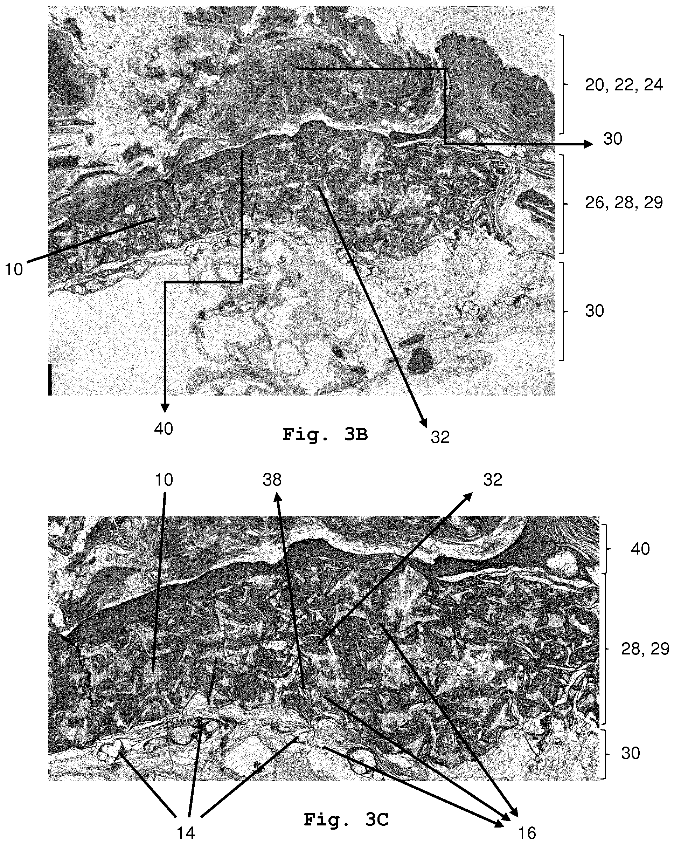

Figs. 3A to 3C show an enlarged section of the former hernia area 28. Within this area 28, a tissue mass 29 is visible that fills the former hernia and consists at least in part of partly degraded carrier mesh 10. In this mass 29, a population of mesenchymal-like cells (elongated and round cells) 32 arranged in interlacing woven bundles can be seen as well as stellate to spindle shaped fibroblasts 16 with relatively clear border and oval-flat nuclei with fine granular chromatin. The mesenchymal cells 32 are believed to stem from the secretome that was deposited on the carrier mesh together with the fibroblasts 16. Mixed with the mesenchymal cells 32 are collagen fibers 38. Adipose tissue 30 is visible below the mass 29 of fibroblast/collagen/mesenchymal-like cells. Above the mass 29, a region of re-epithelialization 40 is visible that separates the mass 29 from several layers of epithelial cells with orthokeratotic hyperkeratosis and intracorneal pustular re-epithelization. Remains of the support mesh 14 can be observed below the mass 29 at the border of the muscularis area 26 to adipose tissue 30 (Fig. 3C).

Usually, fibroblasts are found in much lower numbers and mostly in the subcutaneous area. The high number and the location of the identified cells strongly implies that these stellate to spindle shaped fibroblast cells 16 originate from the implanted carrier mesh 10.

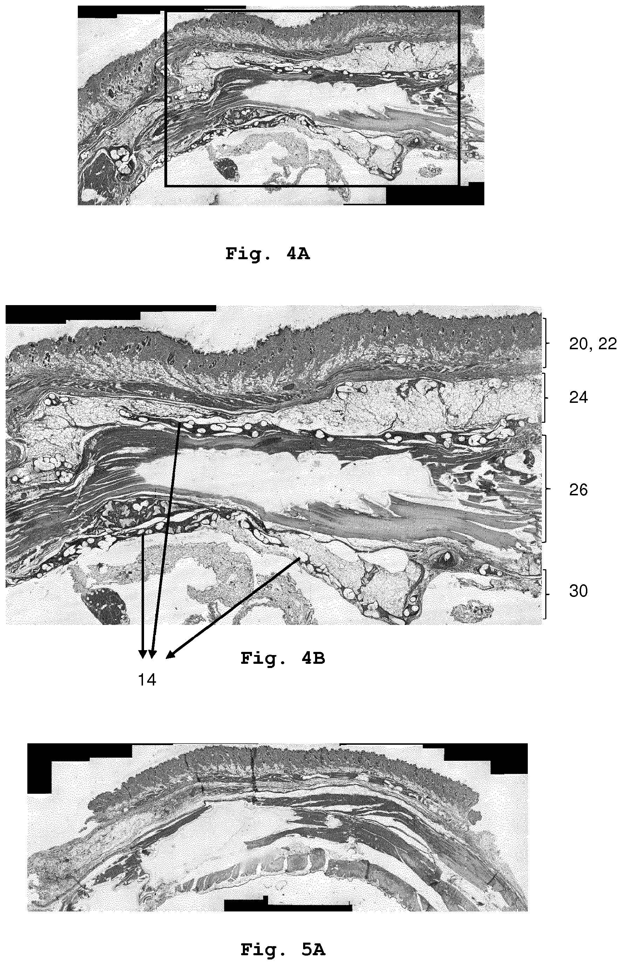

Confirmation of the above findings are given in Figs. 4A-6A: Figs. 4A and 4B show a section through the repaired abdominal wall shown in Fig. 3A-C, yet the cut is not made through the former hernia 28 but adjacent to it. In this adjacent area, only the support mesh 14 is present, since it sized to not only cover the hernia area 28, but also the adjacent areas around it. The support mesh 14 is still clearly visible above and below the muscularis area 26. In contrast to the sections shown in Figs. 3A-3C, no carrier mesh is present.

Fig. 5A, on the other hand, shows a section through tissue at a distance of the hernia area, i.e. through normal tissue of the abdominal wall. As expected, there is neither any support mesh nor any carrier mesh to be seen.

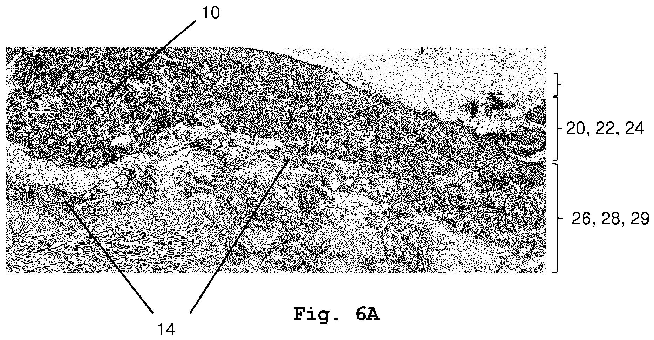

Fig. 6A shows the results from a control experiment, in which a carrier mesh 10 without fibroblasts was implanted to close a soft tissue defect in an abdominal wall of a rat. This control experiment confirmed that the stellate to spindle shaped cells identified in the tissue sections shown in Figs. 3A-3C (and absent in Fig. 6A) are fibroblasts that are not origin of the tissue. Therefore, the histological results confirm that the presence of fibroblasts is not due to normally occurring wound healing and inflammation processes but that the fibroblasts stem from the implantation. In addition, it was seen that the fibroblasts brought into the soft tissue defect with the inventive mesh implant are not only staying at the site of the carrier mesh but are actively migrating into the adjacent host tissue, where they support collagen formation, which is thought to eventually lead to an improved scar formation.

Thus, histological analysis proved the distribution of the implanted fibroblasts not only within the soft tissue defect and within the carrier mesh, but also showed ingrowth and migration of the fibroblasts into the adjacent tissues.

## Claims

1. Biodegradable mesh implant for use in soft tissue repair, in particular surgical hernia or fistula repair, within the body of a patient, comprising
a porous, hydrophilic biodegradable polymeric carrier mesh (10) having a water contact angle of less than 75° and being made of at least a first polymer comprising polylactic acid, and
fibroblasts (16) on or within the polymeric carrier mesh.

2. Implant as claimed in Claim 1, wherein the polymeric carrier mesh has a water contact angle of less than 40°, preferably less than 25°, more preferably less than 15°, most preferably within the range of 0° to 10°.

3. Implant as claimed in Claim 1 or 2, wherein the carrier mesh has a flat sheet-like shape and is elastically deformable to allow folding or rolling thereof.

4. Implant as claimed in one of Claims 1 to 3, wherein the carrier mesh consists of multiple mesh patches, which may be present in a paste-like carrier or in a hydrogel.

5. Implant as claimed in one of Claims 1 to 4, wherein the first polymer consists to at least 70% of poly(lactic acid), more preferably of at least 80% poly(lactic acid) or essentially consists of poly(lactic acid).

6. Implant as claimed in one of Claims 1 to 5, wherein the polymeric carrier mesh comprises or is covered with at least one natural polymer selected from the group consisting of collagen, gelatin, laminin, fibrinogen, albumin, chitin, chitosan, agarose, hyaluronic acidalginate and mixtures thereof, whereby collagen and laminin are preferred.

7. Implant as claimed in one of Claims 1 to 6, wherein the carrier mesh has a total degradation time in the living organism of 1 to 12 months, preferably 1 to 8 months, more preferably about 1 to 6 months, most preferably about 2 to 4 months.

8. Implant as claimed in one of Claims 1 to 7, wherein the fibroblasts are autologous fibroblasts of the patient that is intended to receive the implant.

9. Implant as claimed in one of Claims 1 to 8, further comprising growth factors, preferably selected from the group consisting of interleukins, acidic fibroblast growth factor, basic fibroblast growth factor, epidermal growth factor, insulin like growth factor, insulin like growth factor binding protein, platelet-derived growth factor, transforming growth factor alpha, transforming growth factor beta, VEGF, and HGF.

10. Implant as claimed in Claim 8, wherein the growth factors are growth factors in placental mesenchymal secretome.

11. Method for preparing a mesh implant according to Claim 1 to 10, by
a) providing a biodegradable polymeric carrier mesh comprising polylactic acid;
b) sterilizing the polymeric the polymeric carrier mesh by treating it with H₂O₂ at a temperature below 50°C; and
c) inoculating the polymeric carrier mesh with fibroblasts.

12. Method as claimed in Claim 11, wherein the H₂O₂ treatment in step b) is conducted by treating the polymeric carrier mesh with a H₂O₂ plasma, or by exposing the carrier mesh to a H₂O₂ containing atmosphere, at a pressure within the range of 10⁻² to 10⁻⁶ bar, for at least 2 hours, preferably at least 6 hours, more preferably at least 8 hours, most preferably for about 10 to 12 hours.

13. Method as claimed in Claim 11 or 12, further comprising a step a2) involving plasma cleaning of the carrier mesh before the sterilization step b).

14. Method as claimed in one of Claims 11 to 13, further comprising the step of adding a secretome comprising growth factors is given into the mesh.

15. Implant kit for use in surgical wound repair, in particular fistula or hernia repair, within the body of a patient, the implant kit includes
a mesh implant including a biodegradable polymeric carrier mesh carrying fibroblasts as claimed in one of Claims 1 to 10, and
a polymeric support mesh comprising one or more of the polymers selected from the group consisting of poly(glycolic acid), poly(lactic acid), poly(glycolic acid-lactic acid) and mixtures thereof;
**wherein**
the polymeric carrier mesh has a faster degradation rate than the support mesh.

16. Implant kit as claimed in Claim 15, further including an anti-adhesion mesh comprising two individual or integral layers consisting of a hydrophilic layer facing the abdominal muscle tissue and having a water contact angle below 60° and a hydrophobic layer having a water contact angle above 90°.

17. Implant kit as claimed in Claim 15 or 16, wherein the support mesh has a flat sheet-like shape and the polymeric carrier mesh is in the form of a paste or hydrogel comprising the fibroblasts.
